# EUROPEAN PATENT APPLICATION

(11) **EP 1 821 101 A1**
(43) Date of publication of application: **22.08.2007**
(21) Application number: 05793467.1
(22) Date of filing: 17.10.2005
(51) Int. Cl.: G01N 33/53, G01N 37/00

(54) **GENE EXPRESSION LEVEL STANDARDIZATION METHOD, PROGRAM, AND SYSTEM**

(30) Priority: 15.11.2004 JP 2004331148
(71) Applicant: Sony Corporation, Minato-ku Tokyo 108-0075 (JP)
(72) Inventor: OHTO, Yasunori, (JP); ABE, Tomoteru, (JP)
(74) Representative: Robinson, Nigel Alexander Julian
(86) International application number: PCT/JP2005/019033
(87) International publication number: WO 2006/051670

(57) **Abstract**

It is an object to improve the reliability and accuracy of normalization of gene expression levels, which have been measured separately, by providing a method for the acquisition of an index of high reliability and high accuracy as an index for performing the normalization such that the gene expression levels can be compared and verified. Provided is a method for normalizing a gene expression level, which has been measured for an analysis of a gene expression, by using plural gene expression levels measured for an acquisition of an index. The method includes acquiring a correlation among the plural gene expression levels measured for the acquisition of the index, and using the thus-acquired correlation as an index for normalizing the gene expression level measured for the analysis of the gene expression. The correlation function can be obtained by using, as function values, correlations among plural gene expression levels under at least two sets of experimental conditions (numerals 34 to 38), respectively, with respect to the plural gene expression levels gn acquired under the at least two sets of experimental conditions from cells 31 colleted under the at least two sets of experimental conditions, respectively, and selecting a combination of plural ones of the function values such that the plural function values each approximates to a constant value.

## Description

### [Technical Field]

This invention belongs to a technical field relating to the normalization or standardization, analysis, correction and the like of measurement data of gene expression levels as obtained by using a gene expression analysis plate such as a DNA chip.

### [Background Art]

In recent years, the use of DNA chips or DNA microarrays (hereinafter referred to as "DNA chips" in the present application) has become increasingly common. A DNA chip carries a variety and number of DNA oligosaccharides immobilized together as detecting nucleic acids on a surface of a plate. By detecting with a DNA chip hybridizations between detecting nucleic acids immobilized on the surface of a plate and target nucleic acids in sample nucleic acids collected from cells or the like, gene expressions in the collected cells can be analyzed comprehensively.

Keeping in step with improvements relating to the hybridization detection technologies in the analysis of gene expressions by DNA chips, DNA chips are not limited merely to the detection of existence or non-existence of gene expressions but are also becoming possible to perform the quantitative measurement of gene expression levels. For example, the technology that obtains a quantitative value, which indicates a gene expression level, by the quantitative measurement of a fluorescence intensity upon detection of a hybridization has already been used to some extent.

Attempts are, therefore, under way to normalize a quantitative value that indicates a gene expression level. The term "normalization" as used herein means a conversion of a gene expression level into a numerical value that makes it possible to perform a comparison with another gene expression level obtained by another gene expression analysis. Proposed as normalization methods of a gene expression level include, for example, the method that uses a gene expression level of standard cells as an index for normalization, the method that uses as an index for normalization an expression level of a gene which is being expressed in stationary phase, and the method that uses, as an index for normalization, the total of all gene expression levels measured in a gene expression analysis which makes use of a DNA chip.

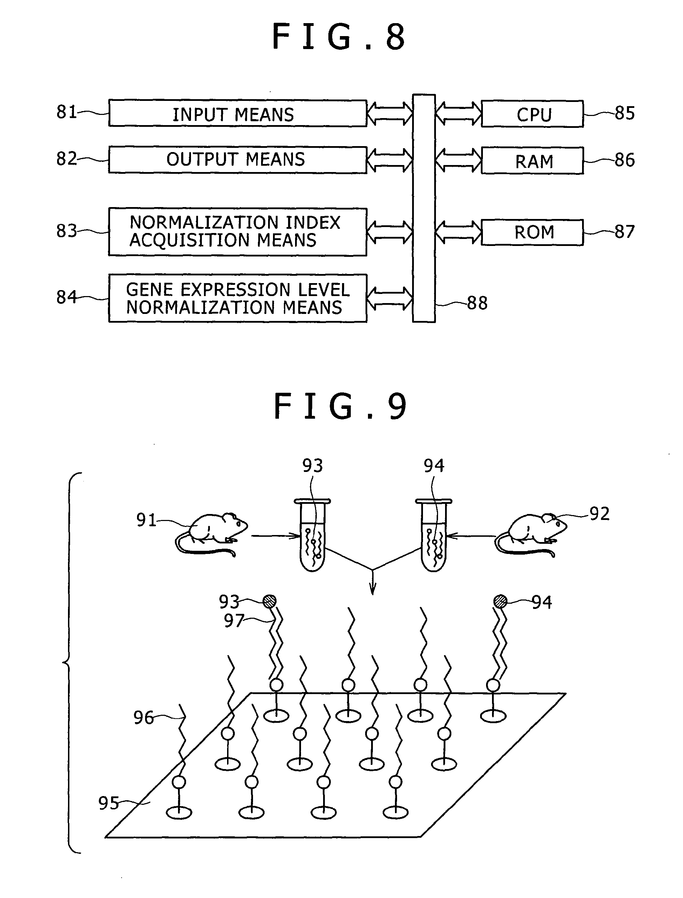

A description will first be made about the method that uses a gene expression level of standard cells as an index for normalization. As illustrated in FIG. 9, sample nucleic acids are obtained from standard cells 91 and cells 92 to be subjected to a gene expression analysis, respectively. After phosphors 93, 94 of different properties are bound to the respective sample nucleic acids, both of the sample nucleic acids are combined together and are then supplied onto a plate surface of a DNA chip. Hybridizations between detecting nucleic acids immobilized on the substrate surface 95 of the DNA chip and sample nucleic acids 97 obtained from the standard cells 91 are next measured in terms of the intensities of fluorescence based on an excitation of the phosphor 93 to acquire a gene expression level of the standard cells. This gene expression level of the standard cells is to be used as an index upon normalizing gene expression levels (intensities of fluorescence based on an excitation of the phosphor 94) relating to the cells 92 to be subjected to the gene expression analysis.

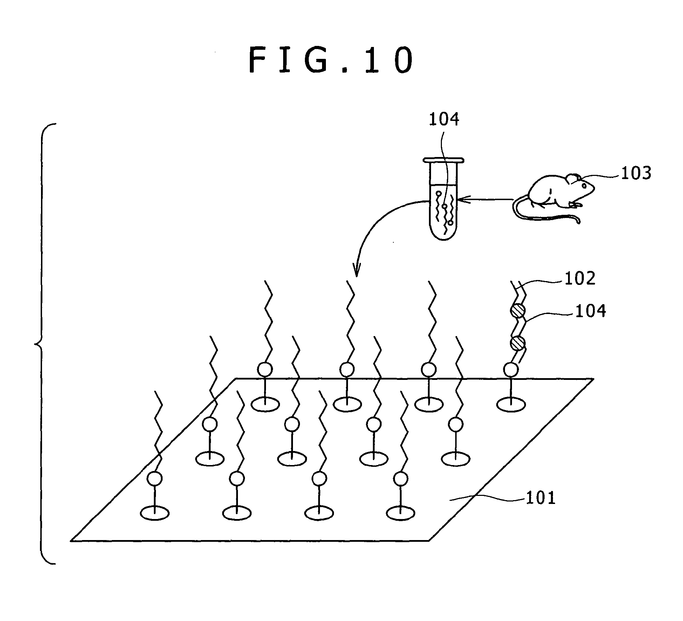

A description will next be made about the method that uses, as an index for normalization, the gene expression level of a gene which is being expressed in stationary phase. As shown in FIG. 10, detecting nucleic acids 102 which are to be subjected to hybridization with the gene, which will be being expressed in stationary phase, are immobilized beforehand on a substrate surface 101 of a DNA chip. By detecting hybridization levels in terms of fluorescence intensity or the like between the detecting nucleic acids 102 and sample nucleic acids 104 collected from cells 103 to be subjected to a gene expression analysis, a gene expression level of the gene in the cells 103 to be subjected to the gene expression analysis is acquired as an index for use upon conducting normalization.

Further, a description will be made about the method that uses, as an index for normalization, the total of all gene expression levels measured in a gene expression analysis which makes use of a DNA chip. This method is based on the rule of thumb that the total of all gene expression levels (fluorescence intensities) measured in a gene expression analysis making use of a DNA chip falls at a substantially constant value, and the total of all the gene expression levels measured in the gene expression analysis making use of the DNA chip is used as an index upon conducting normalization.

In addition, art in the past publications on analysis methods, correction methods and the like for gene expression levels obtained by DNA chips or the like include, for example, Japanese Patent Laid-open No. 2002-71688, JP-A-2002-267668, and Japanese Patent Laid-open No. 2003-28862. "Genetic Algorithms 1 to 4" compiled by KITANO, Hiroaki (Sangyo Tosho Publishing Co., Ltd.) are publications on genetic algorithms which have a relevance to a part of the present invention.

The method that uses a gene expression level of standard cells as an index for normalization is accompanied by a problem in that, except for cases that normalization is conducted using the same standard cells, the respective gene expression levels cannot be compared. Described specifically, when normalization is conducted by using standard cells from a different lot, for example, individual gene expression levels cannot be compared. There is another problem in that each gene expression level can be indicated only in the form of a relative value to the gene expression level of the standard cells.

The method that uses, as an index for normalization, the gene expression level of a gene which is being expressed in stationary phase involves a problem in that it is difficult to find a gene always expressing at a constant level and actually, the gene expression level often varies depending on the collection time of cells or by an external stress applied to the cells. As variations in the index for use in the normalization become substantial, it is difficult to acquire high-reliability numerical values even if gene expression levels are normalized by using, as an index for normalization, the gene expression level of a gene that is being expressed in stationary phase.

The method that uses, as an index for normalization, the total of all expression levels measured in a gene expression analysis which makes use of a DNA chip lacks any theoretical corroboration, and is accompanied by problems in the reliability and accuracy as an index.

A principal object of the present invention is, therefore, to improve the reliability and accuracy of normalization of gene expression levels, which have been measured separately, by providing a method for the acquisition of an index of high reliability and high accuracy as an index for performing the normalization such that the gene expression levels can be compared and verified.

### [Disclosure of Invention]

The present invention provides a method for normalizing a gene expression level, which has been measured for an analysis of a gene expression, by using plural gene expression levels measured for an acquisition of an index, including: acquiring a correlation among the plural gene expression levels measured for the acquisition of the index; and using the thus-acquired correlation as an index for normalizing the gene expression level measured for the analysis of the gene expression.

The correlation can be obtained from a correlation function that includes, as parameters, the plural gene expression levels measured for the acquisition of the index. The correlation function can be obtained, for example, by using, as function values, correlations among plural gene expression levels under at least two sets of experimental conditions, respectively, with respect to the plural gene expression levels acquired under the at least two sets of experimental conditions from cells colleted under the at least two sets of experimental conditions, respectively, and selecting a combination of plural ones of the function values such that the plural function values each approximates to a constant value. The plural gene expression levels acquired under the sets of experimental conditions, respectively, can be obtained, for example, by setting at least two cell collection times.

Further, plural detecting nucleic acids useful for the acquisition of the index can be selected upon acquisition of the correlation function. In this case, it is preferred to select a combination of plural ones of the gene expression levels, the combination having a large degree of contribution to the function values.

As described above, the gene expression level normalization method according to the present invention can normalize a gene expression level, which has been measured for the analysis of gene expression, by plural gene expression levels, which have been measured for the acquisition of an index, and a correlation function. The selection of detection nucleic acids and the acquisition of the correlation function, both of which are used for the acquisition of the index, have to be conducted before the analysis of the gene expression.

It is to be noted that the method according to the present invention can be automatically performed by describing it in the form of a program.

Moreover, the present invention can be implemented in the form of a system. In this case, the system can be built, for example, in a construction provided at least with an input means for inputting the measured gene expression levels, an output means for outputting a correlation function that includes as parameters the plural gene expression levels measured for the acquisition of the index, a normalization index acquisition means for acquiring a correlation among the plural gene expression levels, which were measured for the acquisition of the index and have been inputted by the input means, by arithmetically processing the plural gene expression levels in accordance with the correlation function, and a gene expression level normalization means for normalizing, based on the correlation, the gene expression level measured for the analysis of the gene expression.

The system may be provided with means for obtaining the correlation function by using, as function values, correlations among plural gene expression levels under at least two sets of experimental conditions, respectively, with respect to the plural gene expression levels acquired under the at least two sets of experimental conditions from cells colleted under the at least two sets of experimental conditions, respectively, and selecting a combination of plural ones of the function values such that the plural function value each approximates to a constant value. Further, the system can also be provided with means for selecting the combination of plural gene expression levels, the combination having a large degree of contribution to the function values, as plural detecting nucleic acids useful for the acquisition of the index.

Certain technical terms used herein will be defined hereinafter.

The term "gene expression level" means an expression level of a particular gene in cells, and has a concept that embraces the value of a hybridization level between a detecting nucleic acid immobilized on a plate surface of a DNA chip (measurement data) as measured in terms of fluorescence intensity, an estimated value of a gene expression level as acquired based on the value, and the like.

The term "normalization" means to convert a numerical value, such as fluorescence intensity, which has been obtained by a gene expression analysis or the like, into a numerical value that permits a comparison with all measurement values obtained by other gene expression analyses.

The expression "at least two sets of experimental conditions" means changes in experimental conditions, which with respect to several genes, make their gene expression levels vary. This expression includes, for example, the case that plural sample-cell collection times are set, and the case that the gene expression level is caused to vary by applying predetermined external stimuli to cells. The term "cell collection time" has a concept that a time of acquisition of sample cells is set under the assumption that a time-dependent change in gene expression level has stopped. If it is possible to separately estimate with some certainty a time at which a time-dependent change in gene expression level has stopped, the term "cell collection time" means that time.

The term "hybridization" means a complementary chain (double strand) forming reaction between nucleic acids equipped with complementary base sequence structures.

The term "nucleic acid" means a polymer (nucleotide chain) of the phosphate ester of a nucleoside composed of a purine or pyrimidine base and a saccharide coupled together via a glycosidic linkage, and embraces therein a wide variety of nucleotide chains such as oligonucleotides including probe DNAs, polynucleotides, DNAs (whole lengths and their fragments) formed of purine nucleotides and pyrimidine nucleotides polymerized with each other, cDNAs (c-probe DNAs) obtained by reverse transcription, RNAs, and polyamide nucleotide derivatives(PNAs).

The term "detecting nucleic acid" means nucleic acid molecules, which exist in an immobilized or free state in a medium stored or held at a reaction area and function as probes for detecting nucleic acid molecules having a complementary base sequence that the specifically interacts with the nucleic acid molecules. Typical examples include oligonucleotides or polynucleotides, such as DNA probes. The term "target nucleic acid" means one of sample nucleic acids obtained from cells, the one nucleic acid being capable of hybridizing with the detecting nucleic acid.

By the present invention, it is possible to improve the reliability and accuracy of the normalization of a gene expression level. Namely, separately-measured, gene expression levels can be compared and verified by normalizing the gene expression levels in accordance with the present invention upon conducting an analysis of a gene expression with a DNA chip.

### [Brief Description of Drawings]

[FIG. 1]

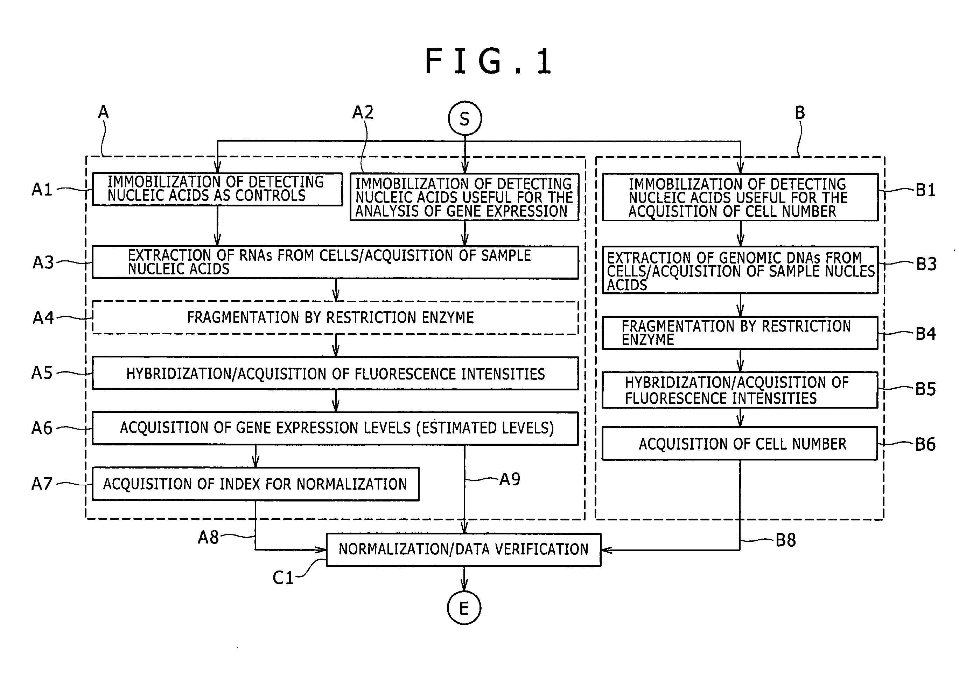

   FIG. 1 is a diagram illustrating a flow of normalization of gene expression levels.
[FIG. 2]

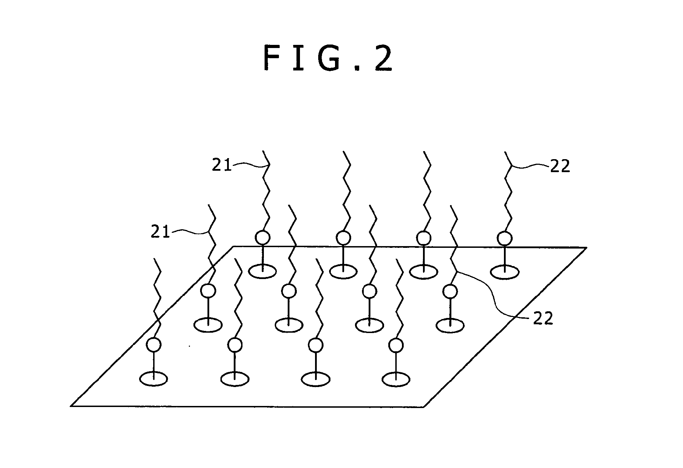

   FIG. 2 is a schematic view showing a construction example of a DNA chip according to the present invention.
[Fig. 3]

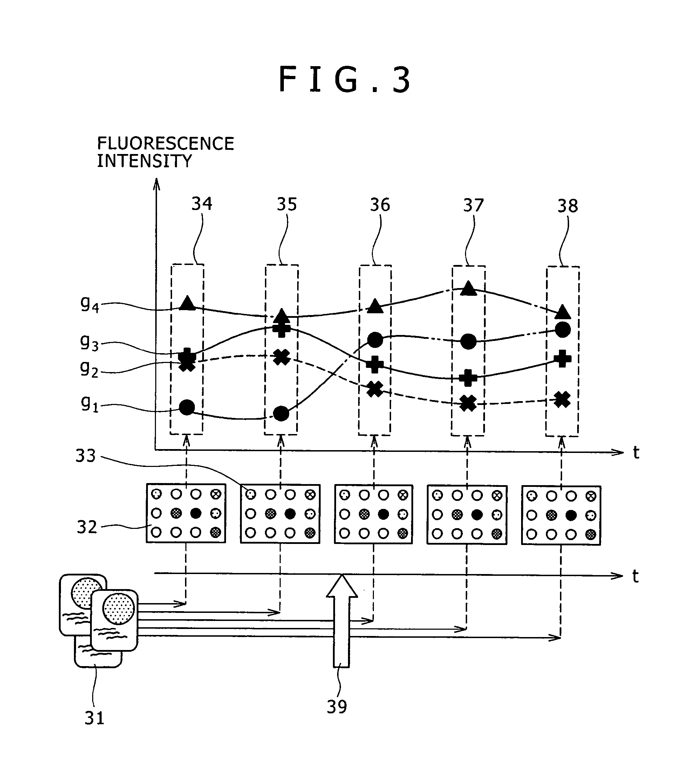

   FIG. 3 is a schematic view depicting one example of a method according to the present' invention for the acquisition of a correlation function.
[FIG. 4]

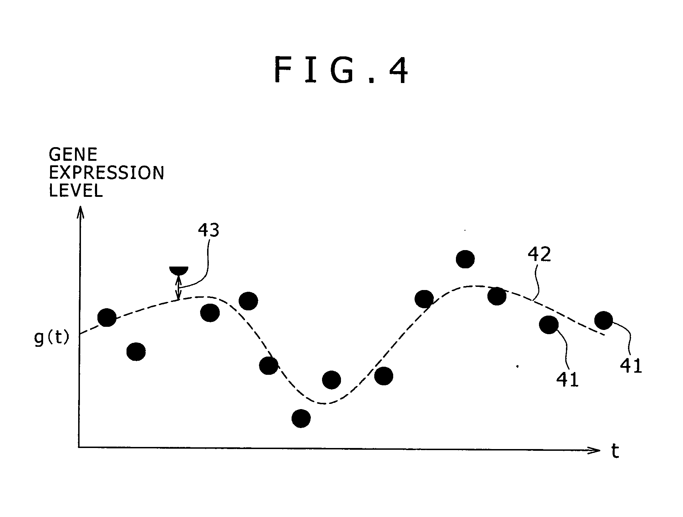

   FIG. 4 is a graph of gene expression levels at plural cell collection times as fitted to a model curve.
[FIG. 5]

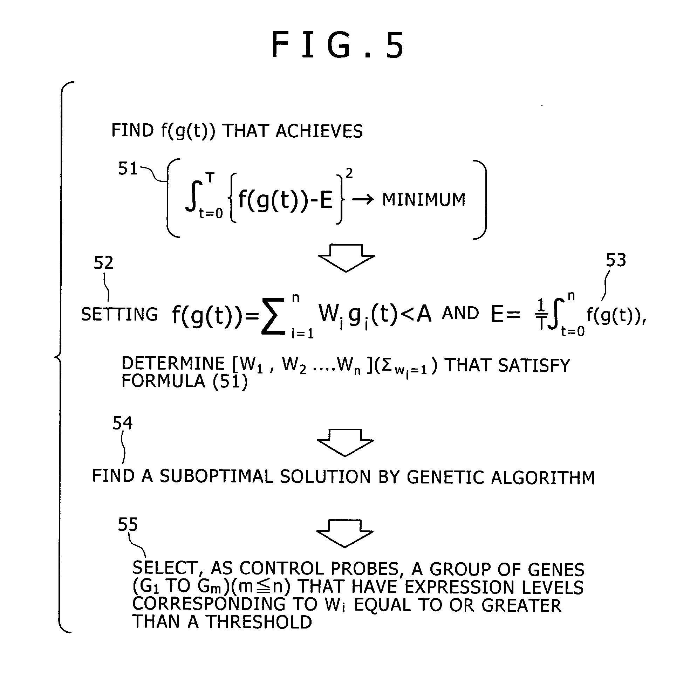

   FIG. 5 is a view showing one example of a selection method of plural detecting nucleic acids useful for the acquisition of an index.
[FIG. 6]

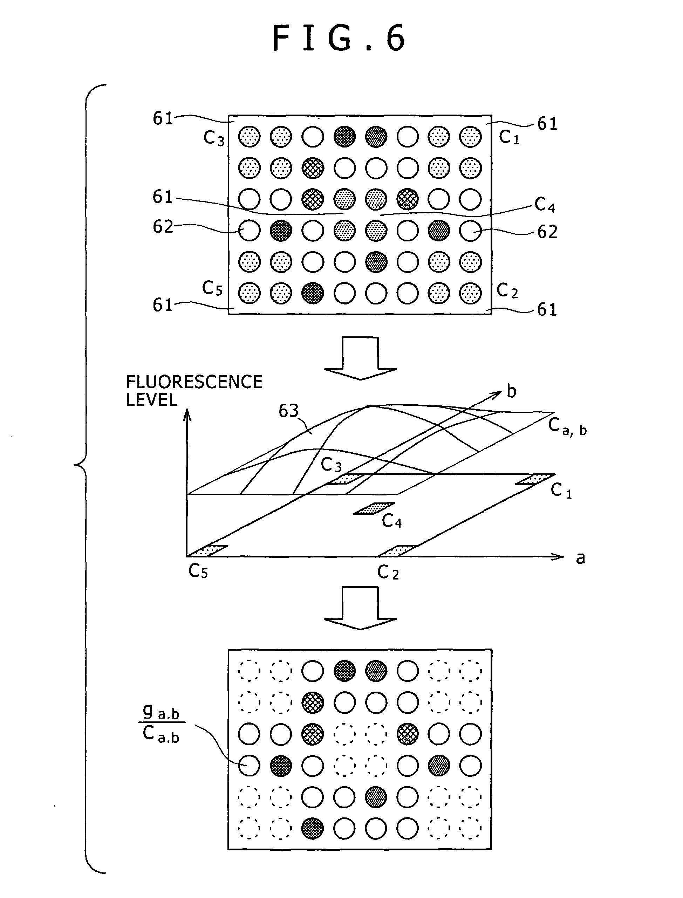

   FIG. 6 is a view showing a method for correcting fluctuations in gene expression levels in a DNA chip.
[FIG. 7]

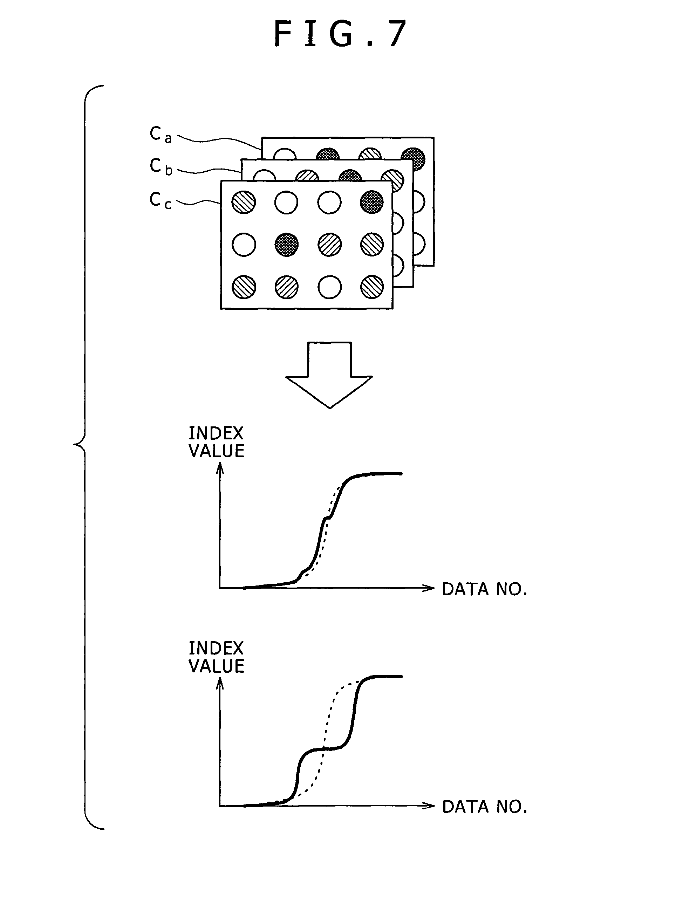

   FIG. 7 diagrammatically illustrates one example of a method for conducting verification of data.
[FIG. 8]
   FIG. 8 is a block diagram depicting one example of a system according to the present invention for normalizing gene expression levels.
[FIG. 9]
   FIG. 9 is a schematic view for describing a conventional technology, and illustrates a method for using a gene expression level of standard cells as an index for normalization.
[FIG. 10]
   FIG. 10 is a schematic view for describing another conventional technology, and illustrates a method for using, as an index for normalization, gene expression levels of genes which are being expressed in stationary state.

### [Best Mode for Carrying out the Invention]

About a preferred embodiment for carrying out the present invention, a description will be made with reference to the accompanying drawings. It is to be noted that the embodiment to be described hereinafter is an exemplification about a case that hybridization levels between detecting nucleic acids immobilized on a plate surface of a DNA chip and target nucleic acids in sample nucleic acids obtained from collected cells were acquired in terms of fluorescence intensity and that the scope of the present invention shall not be narrowly interpreted by the embodiment.

Referring to FIG. 1, a flow of normalization of gene expression levels will be described firstly. In FIG. 1, a flow A indicates an RNA processing flow, and a flow B designates a genomic DNA processing flow. It is to be noted that in FIG. 1, (s) indicates the start of the flow and (E) designates the end of the flow (these meanings will equally apply hereinafter).

The RNA processing flow A is composed of stages (signs A1, A2) of preparing a DNA chip to be used in this flow, stages (signs A3, A4) of obtaining sample nucleic acids from collected cells and preparing them, stages (signs A5, A6) of measuring, in terms of fluorescence intensity, hybridization levels between detecting nucleic acids immobilized on a plate surface of the DNA chip and target nucleic acids in the sample nucleic acids obtained from the collected cells and acquiring gene expression levels, and a stage (sign A7) of acquiring an index useful for the normalization of the thus-measured gene expression levels. These stages will hereinafter be described one after the other.

Concerning the stages (signs A1, A2 in FIG. 1) of preparing the DNA chip, a construction example of the DNA chip for use in the RNA processing flow A will be described with reference to FIG. 2. On the DNA chip to be used in the RNA processing flow A, plural detecting nucleic acids (numeral 21 in FIG. 2) useful for the acquisition of an index and plural detecting nucleic acids useful for an analysis of a gene expression (the nucleic acids other than those indicated at numeral 21 in FIG. 2, for example, those designated at numeral 22) are immobilized beforehand. It is to be noted that the immobilized positions of the plural detecting nucleic acids useful for the acquisition of the index are not limited to the sites depicted in FIG. 2 and, for example, the plural detecting nucleic acids as control probes may be immobilized together at a predetermined position on the plate surface. The selection method of plural detecting nucleic acids to be used in the acquisition of the index will be described subsequently herein.

Referring back to FIG. 1, a description will next be made about the stages (signs A3, A4) of obtaining sample nucleic acids from collected cells and preparing them. In the RNA processing flow A, the sample nucleic acids are obtained by extracting RNAs from the collected cells and synthesizing cDNAs having a complementary sequence to the RNAs in a manner known per se in the art (sign A3). The sample nucleic acids may be fragmented with a restriction enzyme (sign A4).

A description will next be made about the stages (signs A5, A6) of measuring, in terms of fluorescence intensity, the hybridization levels between the detecting nucleic acids immobilized on the plate surface of the DNA chip and the target nucleic acids in the sample nucleic acids obtained from the collected cells and acquiring the gene expression levels. The sample nucleic acids are supplied to the detecting nucleic acids immobilized on the plate surface of the DNA chip, and the hybridization levels between the detecting nucleic acids and the target nucleic acids in the sample nucleic acids are measured by using the fluorescence intensities or the like (sign A5). Based on the measurement data, gene expression levels (estimated levels before normalization) are then acquired (sign A6).

A description will next be made about the stage (sign A7) of acquiring the index for the normalization of the measured gene expression levels. In the stage of sign A7, a correlation among the plural gene expression levels (sign A6) measured for the acquisition of the index is acquired. The thus-acquired correlation is then used as an index for normalizing a gene expression level measured for the gene analysis. This step can be automatically performed by writing it in the form of a program. The term "correlation" as used herein means a value obtained from a correlation function that includes, as parameters, the plural gene expression levels measured for the acquisition of the index. An acquisition method of the correlation function will be described subsequently herein.

Using the index obtained in the stage of sign A7 (arrow A8), gene expression levels (arrow A9) based on the hybridization levels (measurement data) between the plural detecting nucleic acids and the target nucleic acids in the sample nucleic acids are normalized (sign C1). This step can also be automatically performed by describing it in the form of a program.

On the other hand, the genomic DNA processing flow B is composed of a stage (sign B1) of preparing a DNA chip to be used in this flow, stages (signs B3, B4) of obtaining sample nucleic acids from collected cells and preparing them, and stages (signs B5, B6) of measuring, in terms of fluorescence intensity, hybridization levels between detecting nucleic acids for the acquisition of a cell number and target nucleic acids in the sample nucleic acids obtained from the collected cells, and acquiring the number of the collected cells. These stages will hereinafter be described one after the other.

A description will firstly be made about the stage (sign B1) of preparing the DNA chip. On a plate surface of the DNA chip to be used in the genomic DNA processing flow B, detecting nucleic acids useful for the acquisition of the cell number are immobilized beforehand. As the detecting nucleic acids for the acquisition of the cell number, nucleic acids having the same sequence as repeat sequences existing at a substantially constant percentage in a genomic DNA or a part thereof, such as an Alu sequence, are immobilized.

It is to be noted that the detecting nucleic acids for the acquisition of the cell number may be immobilized at any area on the plate surface of the DNA chip. For example, the DNA chip for use in the RNA processing flow A may be provided on the plate surface thereof with an area to immobilize the detecting nucleic acids for the acquisition of the cell number there, or a separate DNA chip may be provided for the acquisition of the cell number.

A description will next be made about the stages (signs B3, B4) of obtaining the sample nucleic acids from the collected cells and preparing them. In the genomic DNA processing flow B, the sample nucleic acids are obtained by extracting the genomic DNAs from the collected cells in a manner known per se in the art (sign B3). The sample nucleic acids extracted from the genomic DNAs are used subsequent to their fragmentation with a restriction enzyme (sign B4).

A description will next be made about the stages (signs B5, B6) of measuring, in terms of fluorescence intensity, the hybridization levels between the detecting nucleic acids for the acquisition of the cell number and the target nucleic acids in the sample nucleic acids obtained from the collected cells and acquiring the cell number of the collected cells. The sample nucleic acids are supplied to the detecting nucleic acids immobilized on the plate surface of the DNA chip, and the hybridization levels between the detecting nucleic acids and the target nucleic acids in the sample nucleic acids are measured by using the fluorescence intensities or the like (sign B5). By quantitatively measuring in terms of fluorescence intensity or the like the repeat sequences existing in the target nucleic acids, an index which can serve as an indication for the cell number of the collected cells is acquired (sign B6).

By converting the gene expression levels (arrow A9), which have been obtained based on the hybridization levels (measurement data) between the plural detecting nucleic acids useful for the analysis of a gene expression and the target nucleic acids, into values per unit cell number while using the index capable of serving as the indication for the cell number of the collected cells, the gene expression levels are normalized (sign C1). It is to be noted that this step can be automatically performed by describing it in the form of a program.

By comparing the gene expression levels normalized based on the index acquired in the stage of sign A7 with the gene expression levels normalized based on the index acquired in the stage of sign B6 and studying the comparison results, verification of the measurement data can be performed (sign C1). This step can also be automatically performed by describing it in the form of a program.

With reference to FIG. 3 to FIG. 5, a description will next be made about a method for the acquisition of a correlation function useful in the present invention. It is to be noted that this method includes the selection method of plural detecting nucleic acids to be used in the acquisition of an index.

In the present invention, the index for normalizing a gene expression level measured for the analysis of a gene expression can be obtained from plural gene expression levels measured for the acquisition of the index and their correlation. The correlation has to be acquired before the measurement of the gene expression level.

The correlation function can be obtained by using, as function values, correlations among plural gene expression levels under at least two sets of experimental conditions, respectively, with respect to the plural gene expression levels acquired under the at least two sets of experimental conditions from cells colleted under the at least two sets of experimental conditions, respectively, and selecting a combination of plural ones of the function values such that the plural function values each approximates to a constant value. A specific example will hereinafter be described in detail.

It is to be noted that as the plural gene expression levels for use in the acquisition of the correlation function, measurement data publicly available from an online data base (MGED, BodyMap or the like) may be used or the measurement of a hybridization may be separately conducted to acquire measurement data.

FIG. 3 is a schematic view depicting one example of the method according to the present invention for the acquisition of the correlation function.

At each preset time t (five times in FIG. 3), cells 31 are firstly collected, followed by the collection of sample nucleic acids from the cells 31. Next, the sample nucleic acids are supplied into the individual wells 33 of a DNA chip 32, and hybridization levels between detecting nucleic acids immobilized on a plate surface of the DNA chip 32 and target nucleic acids in the sample nucleic acids are measured in terms of fluorescence intensity.

In the graph of FIG. 3, the abscissa represents the cell collection time t and the ordinate the fluorescence intensity (gene expression level). In the graph of FIG. 3, fluorescence intensities (gene expression levels, g(t)) are plotted at every cell collection time t with respect to the genes hybridizations of which were detected on the DNA chip 32 (g₁ to g₄ in FIG. 3).

A correlation function is acquired as will be described hereinafter. Firstly, at the respective cell collection times, plural gene expression levels g(t) (g₁ to g₄ in FIG. 3) are combined (for example, numerals 34 to 38 in FIG. 3) to obtain corresponding function values f(g(t)). A selection is then made of a combination of gene expression levels g(t) that the respective function values f(g(t)) of numerals 34 to 38 most approximate to a constant value. The plural detecting nucleic acids selected as the combination are used for the acquisition of an index, and in addition, a function acquired by the combination is used as a correlation function. It is to be noted that this step can be automatically performed by describing it in the form of a program.

Upon obtaining an index as described above for the normalization of a gene expression level measured for the analysis of a gene, the plural detecting nucleic acids which have been selected by the above-described method and are to be used for the acquisition of the index have to be immobilized beforehand on a plate surface of a DNA chip. Further, a correlation function acquired by the above-described method is used.

It is to be noted that, although the correlation function was obtained by setting the five cell collection times in FIG. 3, the correlation function relating to the present invention can be applied when the expression level of the same gene varies under two or more sets of experimental conditions. For example, not only when two or more cell collection times are set but also when the expression level of the same gene is caused to vary by applying an external stimulus 39 to the cells 31, the resulting gene expression levels can be used as measurement data for the acquisition of a correlation function useful in the present invention.

As the plural detecting nucleic acids for use in the acquisition of the index, a gene the expression level of which varies depending on changes in experimental conditions is preferred accordingly. When experimental conditions are changed by setting two or more cell collection times, for example, a gene the expression level of which varies when the cell collection time is changed, like a clock gene, is suited as plural detecting nucleic acids to be employed as control probes.

FIG. 4 is a graph illustrating one example of a method for having gene expression levels at plural cell collection times fitted to a model curve. In the graph, the abscissa represents the cell collection time and the ordinate the cell expression level.

When gene expression levels at plural cell collection times are fitted to the model curve, for example, it is possible to obtain a function value f(g(t)) at every time within a predetermined range.

Plots 41 in FIG. 4 indicate gene expression levels at the individual cell collection times t. Based on these plots 41, a model curve 42 is fitted.

As the model curve, a B-spline curve can be adopted, for example. The model curve 42 is obtained by adjusting parameters such that errors 43 between the respective plots 41 and the model curve 42 are minimized. Upon obtaining the model curve 42, plural model curves of different dimensions are obtained. By evaluating the dimensions of the model curves in accordance with AIC (Akaike's information criterion), the optimal curve g(t) can be obtained. It is to be noted that this step can be automatically performed by writing it in the form of a program.

FIG. 5 is a flow showing one example of a selection method of plural detecting nucleic acids useful for the acquisition of an index. It is to be noted that this method can be automatically performed by writing it in the form of a program.

If plural gene expression levels g(t) are combined, for example, at every time t within a predetermined range upon acquiring a correlation function, the number of combinations becomes vast. Accordingly, plural detecting nucleic acids to be used in the acquisition of an index are selected by using the flow shown in FIG. 5.

As mentioned above, the plural detecting nucleic acids to be used in the acquisition of the index are selected by a method to be described next. Firstly, the plural gene expression levels g(t) are combined to obtain the respective function values f(g(t)). A selection is then made of a combination of gene expression levels g(t) that the respective function values f(g(t)) most approximate to a constant value E, and from this combination, a correlation function f(g(t)) is found (numeral 51).

In the foregoing, the correlation function f(g(t)) may be obtained by using a weight value Wn and setting the function value f(g(t)) as a formula expressing a weighted average and represented by numeral 52 and the constant value E as a formula expressing a time average and represented by numeral 53, respectively, and finding an optimal solution. It is to be noted that in the formula represented by numeral 52, "gᵢ(t)" indicates the expression level of a given gene i at a predetermined time t among gene expression levels gₙ of plural genes n and "A" indicates a permissible error limit.

If the finding of the optimal solution leads to enormous computational complexity, the correlation function f(g(t)) may be obtained by adopting a genetic algorithm and finding a suboptimal solution (numeral 54). It is to be noted that genetic algorithms are described in detail, for example, in "Genetic Algorithms 1-4 (in Japanese)" compiled by KITANO, Hiroaki (Sangyo Tosho Publishing Co., Ltd.).

In the above case, plural genes corresponding to weight values Wᵢ equal to or greater than a threshold are selected as plural detecting nucleic acids to be used in the acquisition of the index (numeral 55).

With reference to FIG. 6, a description will next be made about a method for correcting fluctuations in gene expression levels in a DNA chip. It is to be noted that this method can be performed automatically by describing it in the form of a program.

Firstly, the plural detecting nucleic acids selected by the above-described method and to be used in the acquisition of the index are immobilized at plural areas 61 on a substrate surface of a DNA chip. In the DNA chip of FIG. 6, the detecting nucleic acids are immobilized at five areas, respectively.

Upon measuring hybridization levels, indices obtained by the above-described method are acquired with respect to the areas 61, respectively (C₁ to C₅ in FIG. 6). The individual indices C₁ to C₅ are fitted to a model curve to obtain a curved correction surface 63. It is to be noted that a three-dimensional graph in the middle of FIG. 6 is a graph representing the position of each well 62 on the DNA chip by an a-b plane and the hybridization level (fluorescence level) in each well 62 by a height.

As the curved model surface, a B-spline curved surface can be adopted, for example. By evaluating the dimension of each curved model surface in accordance with AIC (Akaike's information criterion) upon obtaining the curved model surface, an optimal curved surface can be obtained.

The gene expression level (fluorescence level) g_{a,b} in each well 62 is next normalized by using the index C_{a,b} at the corresponding position on the curved correction surface 63.

In addition, an average of the indices obtained with respect to the individual areas 61 may be used as an index for normalization in the verification of the data.

Referring to FIG. 7, by using the index obtained by the above-described method, a description will next be made about one example of a method for conducting the verification of data. It is to be noted that this method can be automatically performed by describing it in the form of a program.

When the measurement of a hybridization is performed a plurality of times, the reliability of the data of each measurement can be verified by using indices (in FIG. 7, Cₐ, C_{b} and C_{c}) obtained in the individual measurements.

If the resulting curve is in a sigmoidal form as in the graph in the middle of FIG. 7 when the values of the indices obtained in the individual measurements are arranged, for example, in an increasing order, it is indicated that the distribution of the values of the indices follows a Gaussian distribution, thereby verifying that the data of the individual measurements have high reliability.

If the resulting curve is in a multi-peak form as in the graph in the bottom of FIG. 7 when the values of the indices obtained in the individual measurements are arranged, for example, in an increasing order, there is a possibility that the data of the individual measurements have low reliability, thereby making it possible to make a decision such as discarding the data.

With reference to FIG. 8, a description will next be made about one example of a system according to the present invention for normalizing gene expression levels.

In FIG. 8, the system according to the present invention is of a construction equipped with an input means 81 for inputting measured gene expression levels, an output means 82 for outputting a correlation function that includes, as parameters, plural gene expression levels measured for the acquisition of an index, a normalization index acquisition means 83 for acquiring a correlation among the plural gene expression levels, which have been measured for the acquisition of the index, by arithmetically processing the plural gene expression levels, which have been measured for the acquisition of the index, in accordance with the correlation function, a gene expression level normalization means 84 for normalizing a gene expression level measured for an analysis of a gene expression, CPU 85, RAM 86, ROM 87, and a bus 88 connecting the above-described individual modules.

In addition to the above-described elements, the system according to the present invention may also be equipped with means (not shown) for obtaining the correlation function by using, as function values, correlations among plural gene expression levels under at least two sets of experimental conditions, respectively, with respect to the plural gene expression levels acquired under the at least two sets of experimental conditions from cells collected under the at least two sets of experimental conditions, respectively, means (not shown) for selecting the combination of plural gene expression levels, the combination having a large degree of contribution to the correlation values, as plural detecting nucleic acids useful for the acquisition of the index, etc.

### [Industrial Applicability]

By the present invention, quantitative measurement of hybridizations in an analysis of gene expressions, the analysis making use of a gene-expression analyzing plate, can be normalized and made highly accurate. As the measurement values of hybridizations can be normalized, the measurement value from each gene expression analysis can be compared and verified with high accuracy.

The method, program and system according to the present invention can be easily incorporated in a measurement system making use of a DNA chip or the like.

## Claims

1. A method for normalizing a gene expression level, which has been measured for an analysis of a gene expression, by using plural gene expression levels measured for an acquisition of an index, comprising:
acquiring a correlation among said plural gene expression levels measured for the acquisition of said index; and
using the thus-acquired correlation as an index for normalizing said gene expression level measured for the analysis of said gene expression.

2. The method according to claim 1, wherein
said correlation is a value available from a correlation function that comprises, as parameters, said plural gene expression levels measured for the acquisition of said index.

3. The method according to claim 2, wherein said correlation function is a correlation function obtained by
using, as function values, correlations among plural gene expression levels under at least two sets of experimental conditions, respectively, with respect to said plural gene expression levels acquired under said at least two sets of experimental conditions from cells colleted under said at least two sets of experimental conditions, respectively, and
selecting a combination of plural ones of said function values such that said plural function values each approximates to a constant value.

4. The method according to claim 3, wherein said plural gene expression levels acquired under said sets of experimental conditions, respectively, have been obtained by setting at least two cell collection times.

5. The method according to claim 3, wherein said combination of plural gene expression levels, said combination having a large degree of contribution to said function values, are selected as plural detecting nucleic acids useful for the acquisition of said index.

6. The method according to claim 1, wherein said gene expression levels are values obtained by measuring, in terms of fluorescence intensity, hybridization levels between a detecting nucleic acid immobilized on a surface of a plate in a DNA chip and a target nucleic acid hybridized with said detecting nucleic acid.

7. A program for normalizing a gene expression level, which has been measured for an analysis of a gene expression, by using plural gene expression levels measured for an acquisition of an index, comprising the steps of:
acquiring a correlation among said plural gene expression levels measured for the acquisition of said index; and
using the thus-acquired correlation as an index for normalizing said gene expression level measured for the analysis of said gene expression.

8. The program according to claim 7, wherein said program comprises the step of
acquiring said correlation from a correlation function that includes, as parameters, said plural gene expression levels measured for the acquisition of said index.

9. The program according to claim 8, wherein said program comprises a program for a step of obtaining said correlation function by
using, as function values, correlations among plural gene expression levels under at least two sets of experimental conditions, respectively, with respect to said plural gene expression levels acquired under said at least two sets of experimental conditions from cells colleted under said at least two sets of experimental conditions, respectively, and
selecting a combination of plural ones of said function values such that said plural function value each approximates to a constant value.

10. The program according to claim 9, wherein plural gene expression levels acquired by setting at least two cell collection times are used.

11. The program according to claim 9, wherein said program comprises a step of selecting said combination of plural gene expression levels, said combination having a large degree of contribution to said function values, as plural detecting nucleic acids useful for the acquisition of said index.

12. The program according to claim 7, wherein said gene expression levels are values obtained by measuring, in terms of fluorescence intensity, hybridization levels between a detecting nucleic acid immobilized on a surface of a plate in a DNA chip and a target nucleic acid hybridized with said detecting nucleic acid.

13. A system for normalizing a gene expression level, which has been measured for an analysis of a gene expression, by using plural gene expression levels measured for an acquisition of an index, comprising at least:
input means for inputting said measured gene expression levels;
output means for outputting a correlation function that includes as parameters said plural gene expression levels measured for the acquisition of said index;
normalization index acquisition means for acquiring a correlation among said plural gene expression levels, which were measured for the acquisition of said index and have been inputted by said input means, by arithmetically processing said plural gene expression levels in accordance with said correlation function; and
gene expression level normalization means for normalizing, based on said correlation, said gene expression level measured for said analysis of said gene expression.

14. The system according to claim 13, wherein said system is provided with means for obtaining said correlation function by
using, as function values, correlations among plural gene expression levels under at least two sets of experimental conditions, respectively, with respect to said plural gene expression levels acquired under said at least two sets of experimental conditions from cells colleted under said at least two sets of experimental conditions, respectively, and
selecting a combination of plural ones of said function values such that said plural function value each approximates to a constant value.

15. The system according to claim 14, wherein plural gene expression levels acquired by setting at least two cell collection times are used.

16. The system according to claim 14, wherein said system is provided with means for selecting said combination of plural gene expression levels, said combination having a large degree of contribution to said function values, as plural detecting nucleic acids useful for the acquisition of said index.

17. The system according to claim 13, wherein said gene expression levels are values obtained by measuring, in terms of fluorescence intensity, hybridization levels between a detecting nucleic acid immobilized on a surface of a plate in a DNA chip and a target nucleic acid hybridized with said detecting nucleic acid.
